# EUROPÄISCHE PATENTSCHRIFT

(11) **EP 1 153 600 B1**
(45) Veröffentlichungstag und Bekanntmachung des Hinweises auf die Patenterteilung: **19.07.2006**
(21) Anmeldenummer: 01710026.4
(22) Anmeldetag: 11.05.2001
(51) Int. Cl.: A61K 8/14, A61K 8/99, A61Q 17/04

(54) **Lichtschutz- bzw. Pflegezubereitungen mit einem Gehalt an DNA-Reparaturenzymen**
Sunscreen and skincare compositions containing DNA-repairing enzymes
Compositions antisolaires et de soin contenant des enzymes réparatrices d'ADN

(30) Priorität: 12.05.2000 DE 10023261; 12.05.2000 DE 10023257; 12.05.2000 DE 10023150; 12.05.2000 DE 10023989; 12.05.2000 DE 10023262; 12.05.2000 DE 10023253
(43) Veröffentlichungstag der Anmeldung: 14.11.2001
(60) Teilanmeldung: 05026408.4
(73) Patentinhaber: STADA ARZNEIMITTEL AG, 61118 Bad Vilbel (DE)
(72) Erfinder: Hansen, Peter, Dr., 63303 Dreieich (DE); Heppner, Andrea, 61197 Florstadt (DE); Schuman, Christof, 35767 Breitscheid-Erdbach (DE)
(74) Vertreter: Hamm, Volker

(56) Entgegenhaltungen:
- EP-A- 0 707 844
- US-A- 4 464 362
- US-A- 5 352 458
- STEGE, H. ET AL.: "Enzyme plus light therapy to repair DNA damage in ultraviolet-B-irradiated human skin" PROC. NAT. ACC. SCI., Bd. 97, Nr. 4, 15. Februar 2000 (2000-02-15), Seiten 1790-1795, XP002233100
- PATENT ABSTRACTS OF JAPAN vol. 1997, no. 02, 28. Februar 1997 (1997-02-28) & JP 08 266275 A (SHISEIDO CO LTD), 15. Oktober 1996 (1996-10-15)

## Beschreibung

Die Erfindung betrifft Lichtsehutzzubereitungen sowie andere Produkte für die Hautpflege bei Sonneneinwirkung, die einen Gehalt an DNA-Reparaturenzymen aufweisen.

Die schädigende Wirkung des ultravioletten Teils der Sonnenstrahlung auf die Haut ist seit langem bekannt. Zum Schutz der Haut sowohl vor UV-A-Strahlung als auch vor UV-B-Strahlung sind zahlreiche physikalische und chemische Filtersubstanzen entwickelt worden, die die Wirkung der einfallenden Sonnenstrahlen abschwächen.

Physikalische Lichtschutzfilter wirken auf der Haut verteilt wie kleine Spiegel, die die UV-Strahlung reflektieren und streuen.

Durch chemische Lichtschutzfilter wird die auf die Haut auftreffende UV-Strahlung in Wärmeenergie umgewandelt. Chemische Lichtschutzfilter können sowohl lipophile als auch hydrophile Eigenschaften aufweisen und entsprechend dieser Eigenschaften unterscheidet man zwischen öllöslichen und wasserlöslichen Filtern. Da die menschliche Haut sowohl lipophile als auch hydrophile Bereiche aufweist, ist es für eine möglichst flächendeckende Wirkung eines Sonnenschutzmittels vorteilhaft, wenn dieses sowohl öllösliche als auch wasserlösliche Filtersubstanzen enthält.

Physikalische und chemische Filtersubstanzen dienen allein dem Zweck, Sonnenstrahlung möglichst vollständig, in jedem Fall aber in ausreichendem Maße zu reflektieren bzw. zu absorbieren, um Schädigungen der Haut zu verhindem. Obwohl dieser Zweck bei derzeit handelsüblichen Produkten bei sachgemäßer Anwendung in hohem Maße erreicht wird, kann durch ihre Verwendung nicht ausgeschlossen werden, daß dennoch UV-Strahlung an die Haut gelangt und dort Schädigungen hervorruft. Diese Schädigungen können eine einfache Hautreizung, z.B. ein leichter Sonnenbrand sein, können sich aber auch in Zellschädigungen manifestieren. Es ist bekannt, daß vor allem durch UV-B-Strahlung DNS-Schäden hervorgerufen werden, die zu Zellmutationen und einer Immunosuppression in der Haut und somit zu Hautkrebs fuhren. DNS-Schäden in der menschlichen Haut werden durch ein komplexes Zusammenwirken verschiedener Enzyme repariert. Da für diesen Prozeß kein Licht benötigt wird, wird er auch als "dark-repair" bezeichnet. Bei einigen Algen, Beuteltieren und einer Gürteltierart ist vor kurzem ein Reparatursystem für Zellschädigungen entdeckt worden, das unter Einwirkung von Licht unter Beteiligung verschiedener Enzyme funktioniert

Aus den US-Patenten 5 296 231, 5 190 762 und 5 272 079 sowie aus EP 0 423 214 (ent sprechend DE 689 17 729) sind Verfahren zur Isolierung von DNA-Reparaturenzymen bekannt. Da Hautsehadigungen durch UV-Licht in der Epidermis, nahe der unteren Basalzellschicht auftreten, ist es vorteilhaft, die DNA-Repataturenzyme in ein Vehikel zu inkorporieren, das die Enzyme durch die über der Basalzellschicht liegenden Hautschichten (Stratum comeum, Stratum lucidum, Stratum granulosum und Stratum spinosum) zum eigentlichen Wirkort transportieren kann. Als besonders geeignetes Vehikel sind in dem oben zitierten Stand der Technik liposomale Zubereitungen beschrieben worden, bei denen die DNA-Reparaturenzyme in Liposomen eingeschlossen sind. Vorbekannte, üblicherweise im Kosmetik- und Arzneimittelsektor verwendete Liposomen sind für die Verabreichung von DNA-Repamturcuzymen nicht geeignet. Eine optimale Penetration der Liposomen durch die Hautschichten und eine optimale Wirksamkeit der Enzyme werden hingegen dann erzielt, werm die Liposomen mittels reiner Phospholipide gebildet werden. Derartige, DNA-Reparaturenzyme enthaltende Liposomen werden von der Firma AGI DERMATICS kommerziell vertrieben, beispielsweise unter der Handelsbezeichnung "Photosemen ®". Bei diesem Produkt handelt es sich um wie zuvor beschriebene Liposomen, in die ein Extrakt aus dem Meeresorganismus Anacystis nidulans eingeschlossen ist, der einen hohen Gehalt an dem DNA-Reparaturenzym Photolyase aufweist.

Die Struktur und die Wirkungsweise des Enzyms Photolyase sind bekannt. Durch eine Bestrahlung menschlicher Haut mit UVB-Strahlung kommt es zur Ausbildung von Cyclobutanpyrimidindimeren (CPDs), den hauptsächlichen DNA-Photoprodukten, in den Zellkernen, einhergehend mit einer Reihe immunsuppressiver Effekte, z.B. der Unterdrückung einer zellvermittelten Immunantwort, beispielsweise einem allergischen Kantaktenzem oder der Hemmung des immunologischen Schlüsselmoleküls ICAM-1. Durch die von dem Enzym Photolyase umfaßten Cofaktoren FADH und MTHF werden unter Einwirkung von Lichtenergie die Cyclobutanpyrimidindimere gespalten und die DNA wird in ihren Ausgangszustand zurückversetzt.

Da viele DNA-Reparaturenzyme und insbesondere die Photolyase ihre Wirkung unter Einwirkung von Licht entwickeln, wäre es grundsätzlich wünschenswert, sie in Lichtschutzformulierungen oder andere Produkte für die Hautpflege bei Sonneneinwirkung einzuarbeiten, um so bereits frühzeitig die DNA-Reparatur zu bewirken. Aufgrund der für liposomale Zubereitungen von DNA-Reparaturenzymen beobachteten guten Hautpenetration, wäre es wünschenswert, die kommerziell erhältlichen, DNA-Reparaturenzyme enthaltenden Liposomen in solche Zubereitungen zu inkorporieren. Seitens des Herstellers dieser liposomalen Zubereitungen wird jedoch explizit darauf hingewiesen, daß sie verschiedene Inkompatibilitäten zeigen. So sollen sie gemäß einer Produktbroschüre nicht folgenden Bedingungen und Substanzen ausgesetzt werden:
- organischen Lösungsmitteln oder Alkohol
- einer Ölbasis
- 5% Glycerin oder Ethylenglykol
- Tensiden oder Emulgatoren oberhalb deren kritischen Micehbildungskonzantration
- Temperaturen oberhalb von 25°C
- pH-Werten unter 6,0 oder über 8,0
- stark vom physiologisch vorliegenden abweichendem osmotischen Druck und schaumbildenden Prozessen.

Die Tatsache, daß vorbekannte Liposomen für die wirksame Verabreichung von DNA-Reparaturenzymen ungeeignet sind sowie die Inkompatibilitäten der eigens für diesen Zweck entwickelten Liposomen ließen es als ausgeschlossen erscheinen, DNA-Reparaturenzyme enthaltende Liposomen in Lichtschutz- oder Pflegeformulierungen zu inkorporieren, da diese regelmäßig auf Öl-in-Wasser oder Wasser-in-Öl-Emulsionen basieren und darüber hinaus oftmals einen Gehalt an Alkohol und/oder Tensiden bzw. Emulgatoren aufweisen. Ein weiterer Nachteil der vorbekannten liposomalen Zubemitungen liegt darin, daß sie einen hohen Gehalt an DNA-Reparaturenzym enthaltenden Liposomen aufweisen müssen, der bei mindestens 1% liegt gemäß der Produktbroschüre sollen die DNA-Reparaturenzyme enthaltenden Liposomen in Mengen von mindestens 1 Gew.-% in kosmetische Zubereitungen eingearbeitet werden. Die im Stand der Technik bekannten, DNA-Reparaturenzym enthaltenden Zusammensetzungen, weisen ebenso einen Gehalt an Liposomen von mindestens 1 Gew.-% auf. Stege et al. (PNAS, 15. Februar 2005, Bd. 97, Nr. 4, Seiten 1790-1795) beschreibt eine Lotion mit 1 Gew.% an Photolyase enthaltende Liposomen für die topische Anwendung auf UV-B-irritierter Haut, in welche die Photolyase enthaltenden Liposomen ohne den Zusatz eines Sonnenschutzes in ein 1 % Hydrogel eingearbeitet wurden.

Aufgabe der Erfindung ist es, lagerstabile kosmetische Zubereitungen mit einem bei akzeptabler Wirksamkeit verminderten Gehalt an DNA-Reparaturenzym enthaltendem Liposomen anzugeben.

Diese Aufgabe wird durch die im unabhängigen Anspruch definierte Zubereitung gelöst.

Die abhängigen Ansprüche definieren vorteilhafte Ausführungsformen der Erfindung.

In den der vorliegenden Anmeldung zugrundeliegenden Untersuchungen wurde überraschend gefunden, daß sich die an sich bekannten und kommerziell erhältlichen DNA-Reparaturenzyme enthaltenden Liposomen, entgegen den ausdrücklichen Informationen des Herstellers, in Formulierungen auf Basis einer ggf. alkoholischen Öl-in-Wasser-(O/W-) bzw. Wasser-in-Öl-(W/O-) Emulsion oder eines alkoholischen Hydrogels einarbeiten lassen, ohne daß die Liposomen eine Zersetzung erleiden.

Des weiteren hat sich in von der Anmelderin durchgeführten *in-vitro* und *in-vivo* Untersuchungen herausgestellt, daß anmeldungsgemäße Zusammensetzungen auch dann noch Enzymaktivität zeigen, wenn der Gehalt an Reparaturenzym enthaltenden Liposomen bei 0,1 bis 0,75-1,0 Gew-% liegt.

Die erfindungsgemäßen Zubereitungen können als ggf. alkoholische O/W bzw. W/O- . Emulsion, als Hydrodispersionsgel oder als ggf. alkoholisches Hydrodispersionsgel formuliert werden.

Die O/W- bzw. W/O-Emulsionen umfassen grundsätzlich eine Ölphase, Wasser sowie DNA-Reparaturenzym bzw. dieses enthaltende Liposomen und ggf. Alkohol. Die Ölphase kann dabei aus Fetten, Ölen gelösten Wachsen oder sonstigen lipophilen Bestandteilen gebildet werden. Als Öle kennen vorteilhaft Paraffinöl, mittelkettige Triglyceride, wie beispielsweise Myritol 318, Octyldodecanol, Isopropylmyristat, Jojobaöl, Kokosnußöl oder Rhizinusöl enthalten sein. Die wäßrige Phase der erfindungsgemäßen Emulsionen wird üblicherweise aus Wasser, ggf. im Gemisch mit vergälltem Alkohol gebildet,

Die erfindungsgemäßen Hydrodispersionsgele umfassen grundsätzlich eine Ölphase, ein geeignetes Verdickungsmittel, Wasser sowie einen wirksamen Gehalt an DNA-Reparaturenzym bzw. dieses enthaltende Liposomen. Die Ölphase kann wiederum aus Fetten, Ölen, gelösten Wachsen oder anderen lipophilen Bestandteilen gebildet werden, wobei als Öle vorteilhaft die oben in Zusammenhang mit den Emulsionen genannten Öle enthalten sein können.

Die Hydrogele gemäß der Erfindung umfassen neben dem DNA-Reparaturenzym bzw. den dieses enthaltenden Liposomen grundsätzlich Wasser, ein geeignetes Verdickungsmittel sowie ggf.Alkohol. Als Verdickungsmittel kommen für die erfindungsgemäßen Hydrodispersionsgele und Hydrogele alle üblicherweise verwendeten Verdickungsmittel in Betracht, bevorzugt werden jedoch Polyacrylsäurederivate, wie beispielsweise Polyacrylate aus der Gruppe der sogenannten Carbopole, Acrylsäurecopolymere, wie beispielsweise Pemulen TR1 oder Xanthan. Der Gehalt an Verdickungsmittel in den erfindungsgemäßen Gelen beträgt 0,05 bis 20 Gew.-%, bevorzugt 0,1 bis 3 Gew.-% und besonders bevorzugt 0,5 bis 1 Gew.-%.

Als DNA-Reparaturenzym enthaltende Liposomen umfassen die erfindungsgemäßen Zubereitungen bevorzugt die von der Firma AGI DERMATICS angebotenen Liposomen, besonders bevorzugt die unter der Bezeichnung photosomen ® "vertriebenen. Die INCI-Nomenklatur der "Photosomen ® " lautet: "Plankton Extract and Lecithin". Die cuzymhaltigen Liposomen können in einer Menge von 0,05 bis 0,75 Gew.- %, bezogen auf die Gesamtzusammensetzung, enthalten sein.Gemäß einer bevorzugten Ausführungsform sind die enzymhaltigen Liposomen in einer Konzentration zwischen 0,2 bis 0,75 Gew.-%, am meisten bevorzugt in Konzentrationen zwischen 0,25 und 0,5 Gew.-% enthalten.

Des weiteren können die erfindungsgemäßen Zubereitungen auch mindestens einen geeigneten Emulgator umfassen. Überraschend wurde gefunden, daß die DNA-Reparaturenzym enthaltenden Liposomen auch dann stabil formuliert werden können, wenn der Emulgator oberhalb seiner kritischen Micellbildungskonzentration in den erfindungsgemäßen Zubereitungen enthalten ist. Als Emulgatoren werden erfindungsgemäß bevorzugt hautverträgliche Emulgatorsysteme auf Basis von Zuckertensiden, z.B. Polyglyceryl-3-Methlyglucose-Distearat, verwendet, wie sie unter der Handelsbezeichnung" Tego Care" angeboten werden.

Besonders bevorzugt enthalten die erfindungsgemäßen Zubereitungen zusätzlich mindestens einen Lichtschutzniter. Es können öllösliche UV-Filter, wasserlösliche UV-Filter, physikalische Lichtschutzfilter sowie Kombinationen dieser in den erfindungsgemäßen Zubereitungen enthalten sein. Als geeignete öllösliche UV-Filter sind zu nennen: Zubereitungen enthalten sei Als geeignete öllösliche UV-Filter sind zu nennen:
- Ester der Zimtsäure, beispielsweise p-Methoxyzimtsäureisoamylester (Neo Heliopan 1000) oder Methoxyzimtsäureoctylester (Neo Heliopan AV);
- p-Aminobenzoesäuren, ihre Ester und Derivate, beispielsweise 2-Ethylhexyl-p-dimethylaminobenzoat (Escalol 507);
- Benzophenene, beispielsweise Benzophenon-3 (Neo Heliopan BB);
- Benzylideneampherderivate, beispielsweise 4-Methylbenzylidencampher (Eusolex 6300);
- Dibenzoylmethane, beispielsweise Butylmethoxydibenzoylmethan (Parsol 1789);
- Octyltriazon (Uvinul T 150) und
- Octocrylene (Neo Heliopan 303).

Die genannten öllöslichen UV-Filter können in Mengen zwischen 1 und 10 Gew.-% in den erfindungsgemäßen Zubereitungen enthalten sein. Als geeignete wasserlösliche UV-Filter sind die 2-Phenylbenzimidazol-5-sulfonsäure (Neo Heliopan Hydro) und deren Salze sowie Benzophenone, wie beispielsweise Benzophenon-4 (Uvinul MS 40) zu nennen. Diese Filtersubstanzen können in Mengen von 1 bis 10 Gew.-% in den erfindungsgemäßen Zubereitungen enthalten sein.

Neben den vorgenannten chemischen Filtersubstanzen können die erfindungsgemäßem Zubereitungen zusätzlich physikalische Lichtschutzfilter in Mengen von 1 bis 10 Gew.-% enthalten, wie beispielsweise Titandioxid oder Zinkoxid. Dabei ist es besonders vorteilhaft, wenn die physikalischen Lichtschutzfilter in hydrophober Form vorliegen, d.h. daß die oberflächig wasserabweisend ausgestaltet sind. Dies kann z.B. dadurch erfolgen, daß die Pigmentteilchen mit einer hydrophoben Oberflachenschicht aus Silikon überzogen werden. Derartige Pigmente sind kommerziell erhältlich, beispielsweise von der Firma Tayca unter der Handelsbezeichnung MT 100 T.

Die folgenden Beispielrezepturen verdeutlichen die Erfindung, wobei die Inhaltsstoffe gemäß der INCI-Nomenklatur bezeichnet werden.

### Beispiel A. Nachweis der Photolyaseaktivität in-vitro und in-vivo

Für die *in-vitro* Untersuchungen wurde ein in Vorarbeiten etabliertes photoimmunologisches Read-Out-System verwendet, in dem es durch die Ausbildung von CPDs in der DNS von menschlichen Hautzellen (Fibroblasten) oder Antigen-präsentierenden Zellen (Monozyten) zu einer Hemmung der Expression des immunologischen Schlüsselmoleküls ICAM-1 kommt. Werden die UVB-bestrahlten Zellen jedoch nach der Bestrahlung mit Photosomen behandelt, dann kann die Hemmung der ICAM-1 Expression durch die mit der Photosomenbehandlung einhergehende Reparatur der CPDs verhindert werden. Somit ist die Verhinderung dieses UVB-induzierten immunsuppressiven Effektes ein Maß für die Aktivität der sich in einer gegebenen. Präparation befindenden Photosomen. In den *in-vitro* Untersuchungen wurden zudem zunächst mittels Verdünnungsreihen ermittelt, ob und in welcher Konzentration die Präparationen zytotoxisch wirken.

In analoger Weise wurde der Aktivitätsnachweis *in-vivo* geführt. Hierbei wurde ebenfalls die Hemmung der ICAM-1 Expression in UVB-bestrahlter menschlicher Haut, die aus der Generation von CPDs in der DNS der Keratinozyten resultiert, als photoimmunologisches Meßsystem verwendet Die zu untersuchenden Phatosomen-haltigen Präparationen wurden topisch auf die bestralten Hautareale aufgetragen und ihre Fähigkeit, die Hemmung der ICAM-1 Expression zu verhindem, als Maß für die in diesen Präparationen enthaltene Photolyaseaktivität genomaen.

Die *in-vitro* und *in-vivo* Untersuchungen wurden mit Zubereitungen folgender Zusammensetzung durchgeführt:

| **Inhaltsstoff** | **Gehalt (Gew.-%)** |
|---|---|
| Pemulen TR1 | 0,5 |
| NaOH | 0,19 |
| Natrium-EDTA | 0,1 |
| Panthenol | 1,0 |
| Glycin | 1,0 |
| Phenoxyethanol | 1,0 |
| Tocopherol | 0,2 |
| Photosom | vanabel |
| Wasser | ad 100 |

Der Gehalt an Photosomen (in Liposomen eingeschlossene Photolyase) betrug 0,1, 0,25, 0,5 Gew.-%.

Die so hergestellten Lösungen mit Photosomengehalten von 0,1, 0,25 und 0,5 Gew.-% wurde den oben beschriebenen *in-vitro* und *in-vivo* Untersuchungen unterzogen und ihre Photolyaseaktivität bestimmt. Als Standard diente eine Photosomen-Zubereitung mit einem Phatosomengehalt von 1 Gew.-%. Für alle drei untersuchten Zusammensetzungen konnte sowohl *in-vivo,* als auch *in-vivo* eine nahezu 100 %-ige Photolyaseaktivität nachgewiesen werden.

## Patentansprüche

1. Stabile Sonnenschtzzubereitung, umfassend einen Gehalt an DNA-Reparaturenzym enthaltenden Liposomen von 0,05 bis 0,75 Gew.-%.

2. Zubereitung nach Anspruch 1,
**dadurch gekennzeichnet, daß** das Reparaturenzym Photolyase ist.

3. Zubereitung nach Anspruch 1,
**dadurch gekennzeichnet, daß** das Reparaturenzym Endonuklease ist.

4. Zubereitung nach einem der vorstehenden Ansprüche,
**dadurch gekennzeichnet, daß** sie als Hydrodispersionsgel oder Hydrogel formuliert ist.

5. Zubereitung nach einem der Ansprüche 1 bis 3,
**dadurch gekennzeichnet, daß** sie einen Träger auf der Basis einer gegebenenfalls alkoholischen Öl-in-Wasser-bzw. Wasser-in-Öl-Emulsion und zusätzlich mindestens einen Emulgator umfasst.

6. Zubereitung nach Anspruch 5,
**dadurch gekennzeichnet, daß** der Emulgator oberhalb seiner kritischen Micollbildungskonzentration enthalten ist.

## Claims

1. Stable sun protection preparation, including a liposome content of 0.05 to 0.75 wt.% containing a DNA repair enzyme.

2. Preparation according to claim 1,
**characterised in that** the repair enzyme is photolyase.

3. Preparation according to claim 1,
**characterised in that** the repair enzyme is endonuclease.

4. Preparation according to one of the preceding claims,
**characterised in that** it is formulated as a hydrodispersion gel or hydrogel.

5. Preparation according to one of claims 1 to 3,
**characterised in that** it includes a carrier based on an oil-in-water or water-in-oil emulsion optionally containing alcohol and additionally at least an emulsifier.

6. Preparation according to claim 5,
**characterised in that** the emulsifier is contained above its critical micelle concentration.

## Revendications

1. Composition stable de protection solaire qui contient une teneur de 0,05 à 0,75% en poids en liposomes qui contiennent une enzyme de réparation de l'ADN.

2. Préparation selon la revendication 1, **caractérisée en ce que** l'enzyme de réparation est la photolyase.

3. Préparation selon la revendication 1, **caractérisée en ce que** l'enzyme de réparation est l'endonucléase.

4. Préparation selon l'une des revendications précédentes, **caractérisée en ce qu'**elle est formulée sous la forme d'un gel hydrodispersé ou d'un hydrogel.

5. Préparation selon l'une des revendications 1 à 3, **caractérisée en ce qu'**elle comporte un porteur à base d'une émulsion éventuellement alcoolisée d'huile dans l'eau ou d'eau dans l'huile et au moins un émulsifiant.

6. Préparation selon la revendication 5, **caractérisée en ce qu'**elle contient l'émulsifiant au-delà de sa concentration critique de formation de micelles.
